# EUROPÄISCHE PATENTANMELDUNG

(11) **EP 0 628 819 A2**
(43) Veröffentlichungstag der Anmeldung: **14.12.1994**
(21) Anmeldenummer: 94108127.5
(22) Anmeldetag: 26.05.1994
(51) Int. Cl.: G01N 33/543, G01N 33/547, C12N 11/14, C07K 17/14, G01N 33/94

(54) **Beschichtete Träger, Verfahren zu ihrer Herstellung und ihre Verwendung zur Immobilisierung von Biomolekülen an Oberflächen von Festkörpern**

(30) Priorität: 08.06.1993 DE 4319037
(71) Anmelder: BAYER AG, 51368 Leverkusen (DE)
(72) Erfinder: Heiliger, Ludger, Dr., D-51373 Leverkusen (DE); Siegmund, Hans-Ulrich, Dr., Elkhart, IN 46516 (US)

(57) **Zusammenfassung**

Neue beschichtete Träger bestehen aus einem inerten Festkörper und einer monomolekularen Schicht eines Polymer, das Aktivgruppen enthält, die am Ende einer hydrophilen Spacergruppe sitzen. Diese Aktivgruppen sind zur Bindung von Molekülen biologischen Ursprungs geeignet. Zwischen dem inerten Festkörper und der monomolekularen Polymerschicht können 0-200 monomolekulare Schichten ohne Aktivgruppen eingeschoben sein. Die Anbringung aller Schichten erfolgt durch LB- oder SA-Technik. Moleküle biologischen Ursprungs können an diese beschichteten Träger angebunden (immobilisiert) werden.

## Beschreibung

Die vorliegende Erfindung beschreibt beschichtete Träger und ein Verfahren zu ihrer Herstellung, die zur Immobilisierung von Proteinen und anderen Biomolekülen, insbesondere von Rezeptorproteinen geeignet sind. Dies ermöglicht eine verbesserte Herstellung von Biosensoren und Biotests.

Die Immobilisierung von Proteinen und anderen Biomolekülen ist für viele analytische und biotechnologische Verfahren von Wichtigkeit. Als analytische Verfahren seien hier insbesondere Festphasen-Immunoassays sowie Bio- und Immunosensoren genannt, bei denen sich durch die Immobilisierung eine Verbesserung der Handhabbarkeit von Immunglobulinen (Antikörpern) ergibt. Nach dem derzeitigen Stand der Technik werden Proteine meist adsorptiv über ionische oder hydrophobe Wechselwirkungen an Oberflächen gebunden oder sie werden über kovalente Bindungen über die Verwendung von Hilfsreagenzien angekoppelt. Als ein mittlerweile klassisch zu nennendes Beispiel für letztere Vorgehensweise sei die Aktivierung von Glas durch APTS (3-Aminopropyl-triethoxysilan) und Glutardialdehyd mit anschließender Bindung von Protein und einer ggf. daran anschließenden Reduktion der entstehenden Schiff-Base durch Natriumborhydrid zu nennen. Einen Überblick über bei Immunoassays benutzte Verfahren findet man beispielsweise in P. Tijssen, "Practice and Theory of Enzyme Immunoassays", S. 297-328 (Elsevier, Amsterdam 1987). Für biotechnologische Verfahren sind daneben Einkapselungsverfahren für Enzyme in permeablen Polymeren oder Membranen gebräuchlich.

Während die adsorptiv arbeitenden Verfahren den Nachteil der mangelnden Stabilität der Proteinimmobilisierung besitzen, erfordert die kovalente Anbindung mit Kupplungs- oder Aktivierungsreagenzien oft eine relativ hohe Anzahl von Prozeßschritten, die Verwendung von hochreinen und instabilen Reagenzien oder die Anwendung von nicht mit allen Proteinen kompatiblen Umsetzungsbedingungen. Auch ist der Wirkungsgrad der Proteinimmobilisierung oft mangelhaft, entweder durch Proteindenaturierung oder durch eine zu geringe Belegung der Oberfläche mit Proteinen. Weiterhin sind einige Aktivierungsreagenzien zur Quervernetzung fähig, wobei sich schlecht definierte Oberflächen ergeben. Die Reproduzierbarkeit der Immobilisierung wird damit ebenfalls sehr schlecht.

In der vorliegenden Erfindung werden diese Probleme dadurch gelöst, daß neue beschichtete Träger, die mit einem dünnen, filmbildenden Polymer überzogen sind, bereitgestellt werden, bei denen das Polymer
a) auf der Oberfläche eines inerten Festkörpers fest anhaftet und
b) Reaktivgruppen zur kovalenten Bindung des zu immobilisierenden Proteins besitzt.

Der Vorteil besteht in einer Reduzierung des Immobilisierungsaufwandes auf im wesentlichen zwei Schritte:
a) Beschichtung des Festkörpers
b) kovalente Anbindung des Proteins an den beschichteten Festkörper
Der Prozeß der Filmbildung (Schichtbildung) kann auf verschiedene Weisen vor sich gehen. Es bieten sich die Langmuir-Blodgett- (LB-) Technik, die Self-Assembly-(SA-) Technik oder eine Kombination aus beiden an. Beide Verfahren sind in A. Ullman, "An Introduction to Ultrathin Organic Films", Part Two, S. 101-132 bzw. Part Three, S. 237-304 (Academic Press, San Diego, 1991) beschrieben. Insbesondere das in DE-OS 40 26 978 und DE-OS 42 08 645 beschriebene SA-Verfahren, bei dem Schichten aus funktionalisierbaren Polyionen verwendet werden, ist aufgrund seiner einfachen Handhabung und der Möglichkeit, beliebig geformte Festkörper sowie Hohlräume zu beschichten, für diesen Zweck gut geeignet.

Die Erfindung betrifft somit beschichtete Träger, bestehend aus
a) einem inerten Festkörper und
b) einer monomolekularen Schicht eines für die Langmuir-Blodgett-(LB-) oder für die Self-Assembly-(SA-) Technik geeigneten Polymers, wobei
c) das Polymer 0,8-20 Mol-%, bevorzugt 2,5-10 Mol-%,bezogen auf die Molzahl aller Comonomere, an einem Comonomer enthält, das eine zur Bindung von Molekülen biologischen Ursprungs geeignete, am Ende einer hydrophilen Spacergruppe sitzende Aktivgruppe besitzt und wobei
d) zwischen dem inerten Träger a) und der monomolekularen Schicht b) 0-200 monomolekulare Schichten eines für die LB- oder SA-Technik geeigneten Polymers ohne Aktivgruppen gemäß c) angeordnet sind.

Die Erfindung betrifft weiter ein Verfahren zur Herstellung beschichteter Träger, dadurch gekennzeichnet, daß ein inerter Träger nach der Langmuir-Blodgett (LB)- oder nach der Self-Assembly (SA-)-Technik mit einer monomolekularen Schicht aus einem für diese Techniken geeigneten Polymer beschichtet wird, wobei dieses 0,8-20 Mol-%, bezogen auf die Molzahl aller Comonomere, an einem Comonomer enthält, das eine zur Bindung von Molekülen biologischen Ursprungs geeignete, am Ende einer hydrophilen Spacergruppe sitzende Aktivgruppe besitzt und wobei zwischen dem inerten Träger und der monomolekularen Schicht 0-200 monomolekulare Schichten eines für die LB- oder SA-Technik geeigneten Polymers ohne die Aktivgruppen enthaltenden Comonomeren angeordnet werden.

Die Erfindung betrifft schließlich die Verwendung des beschichteten Trägers zur Anbindung (Immobilisierung) von Molekülen biologischen Ursprungs.

Der inerte Festkörper kann dabei auch zunächst mit einer oder mehreren Lagen von filmbildenden Substanzen grundiert sein, um eine bessere Anhaftung der obersten, proteinbindenden Schicht zu gewährleisten. Bei Verwendung der LB-Technik sollte der Festkörper eine planare Struktur und eine möglichst einheitliche Oberflächenpolarität besitzen. Für die Beschichtung mit Polyionen nach der SA-Technik bieten sich oberflächlich geladene Materialien an, was sich auch nachträglich einführen läßt, wie z.B. bei oberflächenoxidierten Kunststoffen.

Besonders interessant als inerte Festkörper sind alle Materialien, auf denen Biotests ausgeführt werden oder aus denen die Oberflächen von Biosensoren hergestellt werden. Biotests finden beispielsweise auf Tüpfelplatten, Teststreifen oder in Durchflußsystemen statt; typische Materialien sind Kunststoffe, modifizierte Biopolymere und Gläser. Biosensoren lassen sich nach zahlreichen Verfahren aufbauen. Je nach Nachweismethode (elektrochemisch, optisch oder andere) sind Metallelektroden, Halbleiteroberflächen, membranüberzogene Elektroden, lichtleitende oder lichtdurchlässige Materialien (Gläser, Kunststoffe), Schwingquarze oder Oberflächenresonatoren als erfindungsgemäß einzusetzende Oberflächen zu modifizieren.

Als Reaktivgruppen kommen aus der klassischen Proteinimmobilisierung bekannte Gruppen in Frage, beispielsweise N-Hydroxysuccinimidgruppen, Isocyanat, Isothiocyanat oder Nitrophenylester zur NH₂-Immobilisierung, Iodacetamid- oder Maleimidgruppen zur SH-Immobilisierung oder Hydrazidgruppen zur Bindung von durch Periodatbehandlung gespaltenen Glykoproteinen.

Eine direkte Ankopplung ans Polymerrückgrat, beispielsweise der Einbau von N-Hydroxysuccinimidyl-methacrylat, führte jedoch zu keiner zufriedenstellenden Proteinimmobilisierung. Es wurde gefunden, daß es vorteilhaft ist, diese Gruppen über eine hydrophile Spacergruppe ausreichender Länge an das Polymerrückgrat anzubinden, wodurch sich offenbar eine bessere Annäherung der Reaktivgruppe an das zu bindende Protein in wäßrigem Medium ergibt. Bei den Spacergruppen haben sich heteroatomhaltige Kohlenwasserstoffketten von mehr als 4 Atomen, vorzugsweise von 5-30, besonders bevorzugt 7-30, ganz besonders bevorzugt von 13 bis 30 Atomen Länge bewährt, insbesondere Oligoethylenoxidketten. Beispiele für derartige Gruppen sind die folgenden Monomere (I):

CH₂=C(R¹)―R² (I),

in denen
- R¹: für H oder CH₃ steht und
- R²: die Gruppe 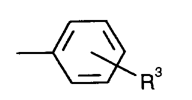 oder -CO-O-R⁴ bedeutet, wobei
- R³: für -(CH₂-)ₘ-SO₂-(CH₂-)ₙ-N=C=X mit X=S oder O oder für -C(CH₃)₂-NH-CO-Y steht,
- R⁴: für -(CH₂-)ₘ-NH-CO-Y, -[CH(R¹)-CH₂-O-)ₒ-SO₂-R⁵ oder -[CH(R¹)-CH₂-O-]ₒ-CO-R⁶ steht,
- Y: für 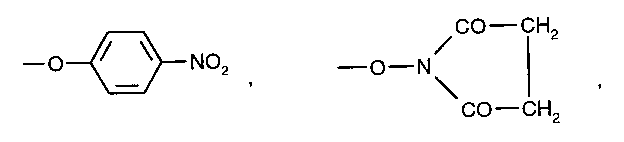 -NH-NH₂ oder -NH-NH₃^{⊕}Cl^{⊖}, 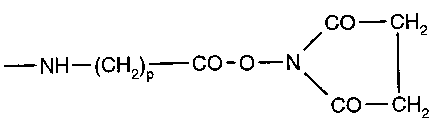 oder -NH-(CH₂-)ₚ-O-SO₂-R⁵ steht,
- R⁵: CH₃, CₚF₂ₚ₊₁, Phenyl oder Tolyl bedeutet,
- R⁶: -(CH₂-)ₘ-CO-Y oder Y bedeutet,
- m und n: unabhängig voneinander ganze Zahlen von 1 bis 4 sind und
- o und p: unabhängig voneinander ganze Zahlen von 1 bis 9 sind.

Bevorzugt als hydrophile Spacergruppen sind Oligoethylenoxidgruppen oder Oligopropylenoxidgruppen mit 5-9 Einheiten.

Diese lassen sich in folgende, beispielhaft genannte, für die LB-Technik geeignete Polymere (II) einbauen: 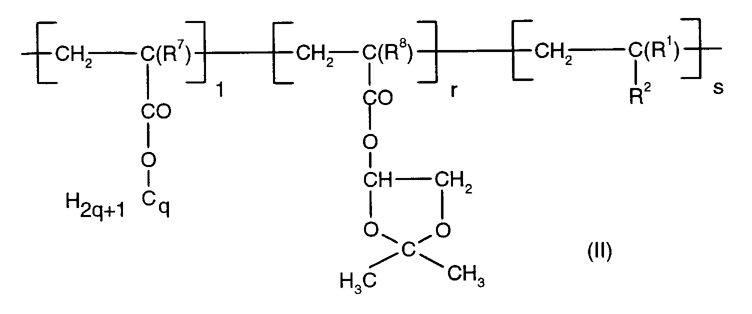
in der
- R¹ und R²: die oben genannte Bedeutung haben,
- R⁷ und R⁸: unabhängig voneinander H oder CH₃ bedeuten,
- q: eine ganze Zahl von 12-22, bevorzugt 16-18 darstellt,
- r: einen Wert von 0,6-1,25, bevorzugt 0,8-1,1, besonders bevorzugt 0,9-1,05 bedeutet und
- s: einen Wert von 0,02-0,4, bevorzugt 0,05-0,2 bedeutet.

Zwischen dem inerten Festkörper und der monomolekularen Schicht aus dem Polymer (II) können 0-200, bevorzugt 0-50, besonders bevorzugt 0-20, hochgeordnete Polymerschichten eingelagert werden, deren Polymer aus dem der Formel (II) durch Fortlassen des Anteils an Monomer mit dem Index s ableitbar ist.

Für die Durchführung der SA-Technik kommen Polymere in Frage, die mehr als eine elektrische Ladung tragen, die demnach Polyionen darstellen.

Als Polykationen kommen beispielsweise Verbindungen mit Aminofunktionen und Sulfoniumgruppen in Betracht, wie Polylysin, Polyallylamin, Polyethylenimin, Polyvinylpyridin, mit Aminofunktionen modifizierte (Meth-)Acrylate (z.B. Poly-N,N-dimethylaminoethyl-methacrylat) und Dextrane (z.B. DEAE-Dextran) sowie Chitosan. Die Aminoverbindungen können entweder durch einfache Protonierung oder durch Quaternisierung in den ionisierten Zustand übergeführt werden

Als Polyanionen kommen beispielsweise Polycarbonsäuren, Polyphosphonsäuren und Polysulfonsäuren in Betracht. Beispiele hierfür sind Polyglutamat, Poly-(meth-)acrylsäure, Polystyrolsulfonsäure oder Dextransulfat.

In einer ähnlichen Weise, wie sie oben für die LB-Technik beschrieben wurde, können auf einem inerten Träger 0-200 Schichten der zur SA-Technik geeigneten obigen Polyionen angebracht werden. In bekannter Weise sind die anzubringenden Polymere jeweils alternativ gegensinnig geladen; der zu beschichtende Träger ist im allgemeinen vorbehandelt, so daß er zur ersten anzubringenden Polymerschicht ebenfalls gegensinnige Ladungen trägt.

Als abschließende Schicht wird sodann auch bei Anwendung der SA-Technik ein polyionisches Polymer angebracht, das 0,8-20 Mol-%, bevorzugt 2,5-10 Mol-%, bezogen auf die Gesamtzähl aller Comonomere, an Monomereinheiten enthält, die zur Bindung von Molekülen biologischen Ursprungs geeignete, am Ende einer hydrophilen Spacergruppe sitzende Aktivgruppen der oben beschriebenen Art enthält (Formel (I)).

Für den Fall, daß als abschließende Schicht ein Polykation angebracht wird und die kationischen Gruppen Hydrazingruppen sind (-NH-NH₂ oder -NH-NH₂·HCl), können diese Hydrazingruppen sowohl die ionische Bindung zur darunter liegenden polyanionischen Schicht bewirken als auch mit ihrer über diese Bindung hinausgehende Menge die Moleküle biologischen Ursprungs binden. In einem solchen Fall kann also die Aktivgruppe über den erfindungsgemäßen Anteil von 0,8-20 Mol-% hinaus bis zu 100 Mol-% ausmachen.

Somit ergeben sich in Analogie zur obigen Formel (II) für die SA-Technik einsetzbare Polymere für die oberste Schicht der Formel 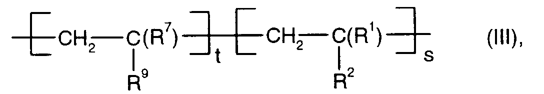
in der R¹, R², R⁷ und s die obige Bedeutung haben,
- t: einen Wert von 1,6 bis 2,25, bevorzugt 1,8 bis 2,1, besonders bevorzugt 1,9 bis 2,05, annimmt und
- R⁹: ein Anion aus der Gruppe von -SO₃^{⊖}, -C₆H₄-SO₃^{⊖}, -COO^{⊖} und -O-PO₃^{⊖⊖} oder
ein Kation aus der Gruppe von 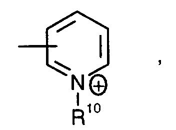 -N(R¹⁰, R¹¹, R¹²), -S(R¹⁰, R¹¹) , 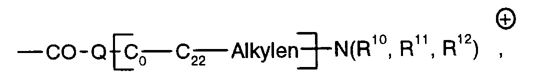 -NH-NH₃^{⊕} oder 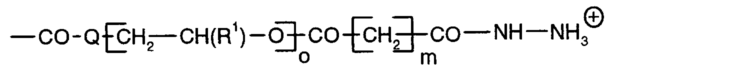 bedeutet,
worin R¹⁰, R¹¹ und R¹² unabhängig voneinander Wasserstoff, C₁-C₂₂-Alkyl, Phenyl oder Benzyl bedeuten und
Q für O oder NH steht und die Indices o und m unabhängig voneinander ganze Zahlen von 1 bis 9 darstellen.

Gegenionen sind bei den Polyanionen H⁺, (Erd)Alkalimetallkationen, NH₄^{⊕} oder ganz oder teilweise durch C₁-C₈-Alkyl, Phenyl oder Benzyl substituiertes Ammonium.

Gegenionen sind bei den Polykationen OH^{⊖}, Halogenid, Sulfat, C₁-C₈-Alkylsulfonat oder Phenylsulfonat. Kationen können auch durch Einwirkung von Wasser oder Säuren erst bei der Anwendung in der SA-Technik entstehen, so beispielsweise aus - NH₂ und Wasser -NH₃^{⊕}OH^{⊖}.

Bevorzugt bei den Polyanionen sind vinylpolymerisierbare Säuren, wie Poly-(meth)acrylsäure oder Polystyrolsulfonsäure. Bei diesen erfolgt der Einbau der Reaktivgruppen ebenso wie bei den Polymeren für die LB-Technik über Reaktivmonomere. Es werden bevorzugt die gleichen Reaktivmonomere (Formel I) zum Aufbau von für die SA-Technik geeigneten Polymeren verwendet.

Bevorzugt bei den Polykationen sind solche mit den im Rahmen der Formel (III) als R⁹ genannten Pyridinium-, Ammonium- oder -CO-Q-Alkylen-ammonium-Kationen.

Der molare Anteil der Reaktivgruppen beträgt zwischen 20 und 0,8 %, bevorzugt zwischen 10 und 2,5 %.

In Analogie zur oben beschriebenen LB-Technik hat bei den 0 bis 200 Schichten unter der obersten Schicht der Index s in (III) den Wert Null; bei den unteren Schichten trägt das Polyanion bzw. Polykation also keine Aktivgruppe. Die Ausnahmestellung der Hydrazingruppe wurde oben bereits beschrieben.

Für die LB-Technik kommen in bekannter Weise Lösungsmittel in Frage, die die Polymeren lösen, aber nicht mischbar sind mit einem Medium, auf dessen Oberfläche die Polymere gespreitet werden. Im wichtigen Fall, bei dem das Medium Wasser ist, sind solche Lösungsmittel z.B. Methylenchlorid und andere halogenierte aliphatische Kohlenwasserstoffe; (Halogen)Kohlenwasserstoffe der aromatischen Reihe; mit Wasser nicht mischbare Ester und andere, dem Fachmann bekannte.

Für die SA-Technik kommen für den Fall von Wasser als Medium hochpolare, gegebenenfalls mit Wasser mischbare Lösungsmittel in Frage, z.B. wasserlösliche Alkohole und Ether, Dimethylsulfoxid, Peralkyl-säureamide und andere, dem Fachmann bekannte.

Moleküle biologischen Ursprungs sind beispielsweise Proteine, Steroide, Stoffwechselprodukte, Vitamine, Nukleinsäuren, bevorzugt biologische Rezeptormoleküle, wie Immunoglobuline, Lectine oder Enzyme, beispielsweise Glukoseoxidase, Urease, Antikörper.

Das zu immobilisierende Protein kann entweder ohne Vorbehandlung, im Falle von Immunglobulinen nach Spaltung zu Fab-Fragmenten oder nach Periodatspaltung von Glykosidresten, sowie über Hilfsproteine, wie im Falle von Immunglobulinen mit Protein A oder Protein G, eingesetzt werden. Mit den so immobilisierten Proteinen können in gleicher Art, wie in EP 429 907-A für Concanavalin A und Mannose beschrieben, Bindungen von Antigen über einen Förster-Energietransfer nachgewiesen werden.

### Beispiele:

### Beispiel 1:

### Synthese des Reaktivpolymers 1 mit Reaktivgruppe 1

In 55 ml Dioxan wurden 4 g Stearylmethacrylat, 2,36 g 4-Methacryloyl-2,2-dimethyl-dioxolan, 0,24 g m-Isopropenyl-α-α-dimethyl-benzyl-isocyanat (m-TMI®, Fa. American Cyanamid Co.) und 10 mg Azobisisobutyronitril eingetragen. Die Apparatur wurde evakuiert, mit Reinststickstoff beschickt, dieser Zyklus zweimal wiederholt und 6 h bei 70^{o}C gerührt. Zu der Rohlösung wurden 0,2 g p-Nitrophenol und 10 mg Diazabicyclo[2,2,2]octan gegeben. Danach wurde die Lösung 16 h bei 55^{o}C gerührt. Nach Erkalten wurde die Rohlösung in 200 ml Methanol eingetropft, wobei ein weißer Niederschlag ausfiel, der abgesaugt wurde.

Ausbeute 5,2 g, entsprechend 76,5 % der theoretischen Ausbeute.

Das ¹H-NMR zeigte die für Polymere typischen breiten Resonanzen, die aufgrund des Gehaltes an m-TMI auch im aromatischen Bereich zwischen 7 und 7,5 ppm liegen, sowie auch die der Esterfunktion benachbarten Protonen zwischen 3,5 und 4,5 ppm und für den Alkylteil die Absorptionen zwischen 0,7 und 2,2 ppm.

Die ¹⁴N - Elementaranalyse ergab 0,4 % N, entsprechend 4 mol-% Reaktivgruppe.

### Beispiel 2:

### a) Herstellung der Reaktivgruppe 2

Unter Stickstoff wurden 5 g 6-Aminohexanol in 95 ml Methylenchlorid gelöst und 6,6 g Isocyanatoethylmethacrylat, in 15 ml Methylenchlorid gelöst, bei Raumtemperatur langsam zugetropft und 2 h nachgerührt. Das Methylenchlorid wurde im Vakuum abgezogen und der Rückstand in Diethylether kristallisiert. Ausbeute 9,8 g, entsprechend 84,5 % der theoretischen Ausbeute.

Das ¹H-NMR zeigte die Harnstoffprotonen bei 5,45 und 5,55 ppm, die vinylischen Protonen bei 5,6 und 6,1 ppm sowie die Alkylresonanzen zwischen 1,2 und 1,7 ppm.

### b) Synthese des Reaktivpolymers 2

In 55 ml Dioxan wurden 4 g Stearylmethacrylat, 2,36 g Methacryloyldimethyldioxolan, 0,32 g Substanz aus Beispiel 2a) und 10 mg Azobisisobutyronitril eingetragen. Die Apparatur wurde evakuiert, mit Reinststickstoff beschickt, dieser Zyklus zweimal wiederholt und 6 h bei 70 ^{o}C gerührt. Die abgekühlte Lösung wurde mit 0,15 g Methylsulfonsäurechlorid und 0,15 g Pyridin versetzt und 2 h bei 40 ^{o}C gerührt. Nach Erkalten wurde die Rohlösung in 200 ml Methanol eingetropft, wobei ein weißer Niederschlag ausfiel, der mit Wasser gewaschen und dann abgesaugt wurde. Ausbeute 5,2 g, entsprechend 76 % der theoretischen Ausbeute.

Das ¹H-NMR zeigte die für Polymere typischen breiten Resonanzen im Alkyl-Bereich zwischen 0,7 und 2,2 ppm sowie auch die der Esterfunktion benachbarten Protonen zwischen 3,5 und 4,5 ppm.

Die ³²S Elementaranalyse ergab 0,45 % S, entsprechend 4,1 mol-% Reaktivgruppe.

### Beispiel 3:

### a) Synthese der Reaktivpolymeren 3 und 4 und der Reaktivgruppe 3

In 15 ml Dioxan wurden je 2,45 g Stearylmethacrylat und Methacryloyldimethyldioxolan sowie 150 mg Azobisisobutyronitril gelöst. Dazu wurden 0,25 g (für Polymer 3) bzw. 0,5 g (für Polymer 4) an Reaktivgruppe 3 (siehe unten; Herstellung beschrieben in Beispiel 1 in DE-OS 42 02 050) zugegeben und 18 h bei 70^{o}C gerührt. Nach dem Erkalten wurden 0,5 g (für Polymer 3), bzw. 1g (für Polymer 4) N,N'-Disuccinimidylcarbonat zugegeben und 5 h bei 50^{o}C gerührt. Die Rohlösung wurde in 500 ml Methanol eingetropft, der entstandene Niederschlag abfiltriert, mit Wasser gewaschen und getrocknet. Das ¹H-NMR zeigte die für Polymere typischen breiten Resonanzen im aromatischen Bereich zwischen 7 und 7,5 ppm sowie auch die der Esterfunktion benachbarten Protonen zwischen 3,5 und 4,5 ppm und für den Alkylteil die Absorptionen zwischen 0,7 und 2,2 ppm. Die ¹⁴N - Elementaranalyse ergab 0,3 % N für Polymer 3, bzw. 0,5 % N für Polymer 4, entsprechend 2,1 mol-%, bzw. 3,6 mol-% N-Hydroxysuccinimid (=NHS)-aktivierten Reäktivgruppe.

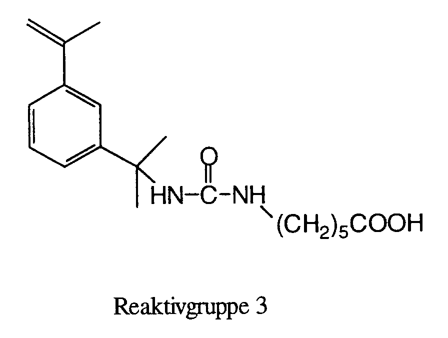

### b) Synthese der Reaktivpolymeren 5 und 6

Der Versuch aus Beispiel 3a) wurde wiederholt, wobei statt Dioxan Dimethylsulfoxid als Lösungsmittel verwendet wurde. Die ¹⁴N-Elementaranalyse ergab 0,4 % N für Polymer 5, bzw. 0,7 % N für Polymer 6, entsprechend 2,8 mol-%, bzw. 5 mol-% der NHS-aktivierten Reaktivgruppe aus Beispiel 3a).

### Beispiel 4:

### Synthese des Reaktivpolymers 7

In 25 ml Dioxan wurden 3,38 g Stearylmethacrylat und 2 g Methacryloyldimethyldioxolan sowie 100 mg Azobisisobutyronitril gelöst. Dazu wurden 0,54 g Reaktivgruppe 3 aus Beispiel 3 gegeben und 20 h bei 70^{o}C gerührt. Nach dem Erkalten wurden 0,2 g NHS und 0,4 g Dicyclohexylcarbodiimid zugegeben und 16 h bei Raumtemperatur gerührt. Die Rohlösung wurde in 400 ml Methanol eingetropft, der entstandene Niederschlag abfiltriert, mit Wasser gewaschen und getrocknet. Das ¹H-NMR zeigte die für Polymere typischen breiten Resonanzen im aromatischen Bereich zwischen 7 und 7,5 ppm sowie auch die der Esterfunktion benachbarten Protonen zwischen 3,5 und 4,5 ppm und für den Alkylteil die Absorptionen zwischen 0,7 und 2,2 ppm. Die ¹⁴N-Elementaranalyse ergab 0,9 % N, entsprechend 6 mol-% der Reaktivgruppe 3 aus Beispiel 3.

### Beispiel 5:

### a) Herstellung der Reaktivgruppe 4

In 40 ml Dioxan wurden 5 g Ethylenoxid-Oligomer-methacrylat mit einem mittleren Mol.-Gew. von 350 (Blemmer PE-350®, Firma Nippon Oil & Fats), 1,13 g Succinanhydrid sowie 10 mg 2,5-Bis-tert.-butylphenol gelöst, 5 Tropfen konzentrierte Schwefelsäure zugegeben und 12 h unter Rückfluß gekocht. Die Schwefelsäure wurde mit Natriummethanolat neutralisiert und die Lösung eingeengt. Der Rückstand wurde mit Methylenchlorid aufgenommen und die Lösung filtriert. Das Filtrat wurde im Hochvakuum eingeengt und der Rückstand getrocknet. Das ¹H-NMR zeigte die vinylischen Protonen bei 5,55 und 6,1 ppm, die Ethylenoxid-Einheiten bei 3,65 ppm, die der Esterfunktion benachbarten Protonen bei 4,25 ppm und die α-Carbonylprotonen bei 2,65 ppm.

### b) Synthese des Reaktivpolymers 8

In 10 ml Dimethylsulfoxid wurden 1,3 g Natrium-p-styrylsulfonat, 0,2 g Reaktivgruppe 4 aus Beispiel 5a) sowie 0,5 g Cumarinfarbstoff 1 (Formel siehe unten; siehe auch Beispiel 2 in DE 4114482 A1) gelöst und nach Zugabe von 20 mg Azobisisobutyronitril 15 h bei 65 ^{o}C gerührt. Die viskose Rohlösung wurde in 100 ml Ethanol eingetropft und der entstandene Niederschlag abgesaugt und in 12 ml Dimethylsulfoxid wieder gelöst. Der Lösung wurden 0,1 g NHS und 0,2 g Dicyclohexylcarbodiimid zugesetzt und 16 h bei Raumtemperatur gerührt. Die Lösung wurde in 100 ml Ethanol eingetropft, der Niederschlag abgesaugt und im Hochvakuum getrocknet. Das ¹H-NMR zeigte die für Polymere typischen breiten Banden mit Ethylenoxid-Einheiten bei 3,65 ppm, aromatischen Absorptionen zwischen 6,5 und 8,5 ppm sowie die aliphatischen Protonen zwischen 0,7 und 2 ppm. Die auf den reinen Farbstoff normierten Extinktionswerte bei 385 nm ergaben 20 Gew.-% Farbstoffanteil im Polymer.

Die ¹⁴N-Elementaranalyse ergab 1,45 % N, entsprechend 4 mol-% NHS-aktivierte Reaktivgruppe 4 aus Beispiel 5a).

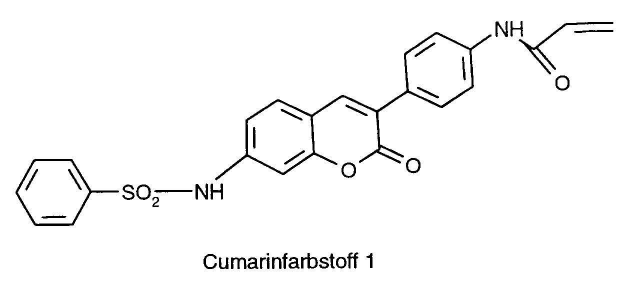

### Beispiel 6:

### Synthese des Reaktivpolymers 9

In 10 ml Dimethylacetamid wurden 2,14 g Stearylmethacrylat, 1,06g Methacryloyldimethyldioxolan, 0,75 g Cumarinfarbstoff 2 (Formel siehe unten; siehe auch Beispiel 3 in DE-OS 42 13 323) sowie 41 mg Azobisisobutyronitril gelöst. Dazu wurden 0,142 g Reaktivgruppe 3 aus Beispiel 3a) gegeben und 20 h bei 70 ^{o}C gerührt. Nach dem Erkalten wurden 49 mg NHS und 88 mg Dicyclohexylcarbodiimid zugegeben und 16 h bei Raumtemperatur gerührt. Die Rohlösung wurde in 400 ml Methanol eingetropft, der entstandene Niederschlag abfiltriert, mit Wasser gewaschen und getrocknet. Das ¹H-NMR zeigte die für Polymere typischen breiten Resonanzen im aromatischen Bereich zwischen 6,5 und 8,3 ppm sowie auch die den Estergruppen benachbarten Protonen zwischen 3,5 und 4,5 ppm und für den Alkylteil die Absorptionen zwischen 0,7 und 2,2 ppm. Die auf den reinen Farbstoff normierten Extinktionswerte bei 480 nm ergaben 15 Gew.-% Farbstoffanteil im Polymer. Die ¹⁴N-Elementaranalyse ergab 1,5 % N, entsprechend 4 mol-% der NHS-aktivierten Reaktivgruppe 3 aus Beispiel 3.

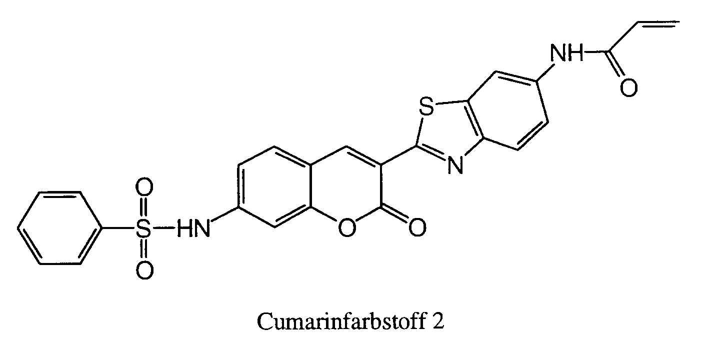

### Beispiel 7:

### a) Herstellung der Reaktivgruppe 5

In 5 ml Acetonitril wurden bei 0 ^{o}C 1 g Natriumcarbonat und 2 g Reaktivgruppe 4 aus Beispiel 5a) vorgelegt und 0,51 g Isobutylchloroformat in 5 ml Acetonitril zugetropft. Es wurde 1 h bei dieser Temperatur gerührt, weitere 10 ml Acetonitril zugesetzt und die Mischung unter Stickstoffatmosphäre filtriert. Das Filtrat wurde bei 0 ^{o}C einer Lösung aus 0,18 g Hydrazinhydrat in 5 ml Acetonitril zugetropft und 2 h bei dieser Temperatur gerührt. Die Lösung wurde filtriert, mit 1 n Salzsäure auf pH 4 gestellt und im Hochvakuum eingeengt, wobei ein gelbes, viskoses Öl zurückblieb.

Das ¹H-NMR zeigte die vinylischen Protonen bei 5,55 und 6,1 ppm, die Ethylenoxid-Einheiten bei 3,65 ppm, die der Esterfunktion benachbarten Protonen bei 4,3 ppm und die α-Carbonylprotonen bei 2,65 ppm. Die ¹⁴N-Elementaranalyse ergab 4,8 Gew.-%, entsprechend 4,88 Mol-% an Reaktivgruppe.

### b) Synthese des Reaktivpolymers 10

In 4 ml Dimethylsulfoxid wurden 0,65 g Natrium-p-styrylsulfonat, 0,1 g Reaktivgruppe 5 aus Beispiel 7a) sowie 0,25 g Cumarinfarbstoff 1 aus Beispiel 5b) gelöst und nach Zugabe von 10 mg Azobisisobutyronitril 15 h bei 65 ^{o}C gerührt. Die viskose Rohlösung wurde in 60 ml Ethanol eingetropft und der entstandene Niederschlag abgesaugt und im Hochvakuum getrocknet. Das ¹H-NMR zeigte die für Polymere typischen breiten Banden mit Ethylenoxid-Einheiten bei 3,65 ppm, aromatischen Absorptionen zwischen 6,5 und 8,5 ppm sowie die aliphatischen Protonen zwischen 0,7 und 2 ppm. Die auf den reinen Farbstoff normierten Extinktionswerte bei 385 nm ergaben 20 Gew.-% Farbstoffanteil im Polymer.

Die ¹⁴N-Elementaranalyse ergab 1,64 % N, entsprechend 4 mol-% Reaktivgruppe 5 aus Beispiel 7a).

### Beispiel 8:

### Synthese des Vergleichspolymers 11:

In 20 ml Dioxan wurden 3,38 g Stearylmethacrylat sowie 2 g Methacryloyldimethyldioxolan mit 100 mg Azobisisobutyronitril gelöst und 20 h bei 70 ^{o}C gerührt. Nach dem Erkalten wurde die Rohlösung in 200 ml Methanol eingetropft, der entstandene Niederschlag abfiltriert, mit Methanol gewaschen und getrocknet. Das ¹H-NMR zeigte die für Polymere typischen breiten Resonanzen der der Esterfunktion benachbarten Protonen zwischen 3,5 und 4,5 ppm und für den Alkylteil die Absorptionen zwischen 0,7 und 2,2 ppm.

### Beispiel 9

### Immobilisierung von β-Galactosidase

Ein Glas- oder Kunststoffträger wurde mit einer LB- (Verfahren nach DE-OS 39 38 598) oder SA-Schicht (Verfahren nach DE-OS 42 08 645) überzogen und mit einem zweiten gleichartigen Träger abgedeckt, dessen Ränder mit einem 0.1 mm starken Teflonband überzogen waren, so daß sich zwischen beiden Trägern 0.1 mm Abstand ergab. Dieser Zwischenraum wurde mit 1 ml einer Lösung von β-Galactosidase (1 mg/ml, 10 mmol Carbonatpuffer pH 8) gefüllt und anschließend 1 h bei Raumtemperatur inkubiert. Die Abdeckung wurde anschließend vom Probenträger entfernt und der Träger dreimal mit EPPS-Puffer (0.1 mol/l N-[2-Hydroxyethyl]-piperazin-N'-[3-propansulfonsäure] (EPPS), 1 mmol/l MgCl₂, 0.05 % NaN₃, 0.1 g/l Rinderserumalbumin, pH 8) gewaschen. Das Plättchen wurde mit 2 ml einer 0.5 mmol/l Lösung von Dimethylacridinon-galactosid (DMAG) in EPPS-Puffer 1 h bei 37°C inkubiert. Anschließend wurde die Lösung in eine Küvette umgefüllt und bei 634 nm im Photometer gegen unbehandelte DMAG-Lösung vermessen. Die Resultate sind in folgender Tabelle dargestellt:

| Substanz | Extinktion (634 nm) |
|---|---|
| Reaktivpolymer 1 | 1.46 |
| Reaktivpolymer 2 | 0.64 |
| Reaktivpolymer 2 | 0.44 |
| Reaktivpolymer 3 | 0.317 |
| Reaktivpolymer 4 | 0.436 |
| Reaktivpolymer 5 | 0.471 |
| Reaktivpolymer 6 | 1.503 |
| Reaktivpolymer 7 | 0.861 |
| Reaktivpolymer 8 | 1.912 |
| Reaktivpolymer 9 | 2.16 |
| Vergleichspolymer 11 | 0.06 (ohne Reaktivgruppe) |
| APTS/GA (s. unten) | 0.363 (Vergleichsprobe, konventioneller Aktivierung) |

Man erkennt gegenüber einer Probe ohne Reaktivgruppen (adsorptive Bindung) und gegenüber einer auf konventionelle Weise mit Aminopropylsilan und Glutardialdehyd (APTS/GA) behandelten Probe eine angehobene Enzymaktivität.

### Beispiel 10

### Immobilisierung von Immunglobulin über Protein A

Eine mit einer LB-Schicht (Substanz: Reaktivpolymer 9) überzogener Streifen aus Polycarbonat-Folie ("Macrofol") wurde mit einem Deckglas abgedeckt, dessen Ränder mit einem 0.1 mm starken Teflonband überzogen warem, so daß sich zwischen beiden Trägern 0.1 mm Abstand ergab. Dazwischen wurden 0.25 ml einer Lösung von Protein A (0.1 mg/ml in 0.01 mol/l Carbonatpuffer pH 8.0) hindurchgegeben und anschließend über Nacht bei Raumtemperatur inkubiert. Anschließend wurde das Deckglas entfernt und die Folie in Citratpuffer (25 mmol/l Citrat, 15 mmol/l Kaliumphosphat, 0.5 mol/l KCl, 1 mmol/l MgCl₂) gespült. Danach wurde mit 0.5 ml einer Lösung von Anti-Digoxin-IgG (0.2 mg/ml in Citratpuffer pH 6.4) benetzt und erneut abgedeckt. Nach 1 h bei Raumtemperatur wurde das Deckglas wiederum entfernt und erneut mit 2ml Citratpuffer pH 6.4 gewaschen, die Folie trocknen gelassen und in kleinere Probenstücke zerschnitten. Diese Proben wurden in eine Lösung von Tetramethylrhodamin-markiertem Digitoxigenin (1 µg/ml in Citratpuffer pH 6.4) eingetaucht und bei 515 und 577 nm die Fluoreszenz gemessen (Anregung: 405 nm). Über das Verhältnis der Fluoreszenzen 577 zu 515 nm (Förster-Energietransfer, wie in DE-OS 39 38 598 beschrieben) ließ sich die Anbindung des Digitoxigeninderivates an immobilisiertes Immunglobulin und dessen Waschstabilität gegenüber Citratpuffer pH 6.4 detektieren (s. Tabelle).

| Typisches Bindungsverhalten von TRITC-Digitoxigenin | |
|---|---|
| | Fluoreszenzverhältnis 577/515 |
| vor TRITC-Digitoxigenin | 0.51 |
| nach TRITC-Digitoxigenin | 1.80 |
| 1x gewaschen | 1.82 |
| 2x gewaschen | 1.50 |
| 3x gewaschen | 1.79 |
| nochmals mit Wasser gewaschen | 2.26 |

### Beispiel 11

### Immobilisierung von Dig-AK über Hydrazidchemie

Ein mit einer SA-Schicht (Reaktivpolymer 10, auf Polylysin-Grundierung) überzogener Streifen aus oberflächenaktivierter PET-Folie (AGFA "gold", 175 µm) wurde mit einem Deckglas abgedeckt, dessen Ränder mit einem 0.1 mm starken Teflonband überzogen waren, so daß sich zwischen beiden Trägern 0.1 mm Abstand ergab. Dieser Zwischenraum wurde mit 1 ml einer Lösung von Anti-Digoxin-IgG versetzt, der zuvor einer Periodatoxidation unterzogen wurde (4 mg IgG in 0.5 ml 0.01 M NaIO₄, 0.1 M Acetatpuffer pH 5.5, 45 min bei Raumtemperatur inkubiert, anschließend über Biogel P6G entsalzt). Nach Inkubieren über Nacht bei 8°C wurde das Folienstück mit Citratpuffer pH 6.4 (siehe Beispiel 10) gespült, trocknen gelassen und in kleinere Stücke zerschnitten. An diesen Probenfolien wurde vor und nach Eintauchen in eine Lösung, die TRITC-markiertes Digitoxigenin (1 µg/ml in Citratpuffer pH 6.4, mit Zusatz von 0.001 Vol-% Tween 20) enthielt, bei 495 und 577 nm die Fluoreszenz gemessen (Anregung: 405 nm). Über das Verhältnis der Fluoreszenzen 577 zu 495 nm ließ sich die Anbindung des Digitoxigeninderivates an immobilisiertes Immunglobulin und dessen Waschstabilität gegenüber Citratpuffer pH 6.4 / Tween 20 detektieren (s. Tabelle).

| Typisches Bindungsverhalten von TRITC-Digitoxigenin | |
|---|---|
| | Fluoreszenzverhältnis 577/495 |
| vor TRITC-Digitoxigenin | 0.12 |
| nach TRITC-Digitoxigenin | 0.62 |
| 1x gewaschen | 0.51 |
| 2x gewaschen | 0.49 |
| 3x gewaschen | 0.52 |
| 4x gewaschen | 0.46 |

## Patentansprüche

1. Beschichteter Träger, bestehend aus
a) einem inerten Festkörper und
b) einer monomolekularen Schicht eines für die Langmuir-Blodgett-(LB-) oder für die Self-Assembly-(SA-) Technik geeigneten Polymers, wobei
c) das Polymer 0,8-20 Mol-%, bevorzugt 2,5-10 Mol-%, bezogen auf die Molzahl aller Comonomere, an einem Comonomer enthält, das eine zur Bindung von Molekülen biologischen Ursprungs geeignete, am Ende einer hydrophilen Spacergruppe sitzende Aktivgruppe besitzt und wobei
d) zwischen dem inerten Träger a) und der monomolekularen Schicht b) 0-200 monomolekulare Schichten eines für die LB- oder SA-Technik geeigneten Polymers ohne Aktivgruppen gemäß c) angeordnet sind.

2. Träger nach Anspruch 1, bei dem das Comonomer nach c) mit der am Ende einer hydrophilen Spacergruppen sitzenden Aktivgruppe eines der Formel
CH₂=C(R¹)―R²
ist, in der
R¹ für H oder CH₃ steht und
R² die Gruppe 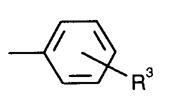 oder -CO-O-R⁴ bedeutet, wobei
R³ für -(CH₂-)ₘ-SO₂-(CH₂-)ₙ-N=C=X mit X=S oder O oder für -C(CH₃)₂-NH-CO-Y steht,
R⁴ für -(CH₂-)ₘ-NH-CO-Y, -[CH(R¹)-CH₂-O-)ₒ-SO₂-R⁵ oder -[CH(R¹)-CH₂-O-]ₒ-CO-R⁶ steht,
Y für 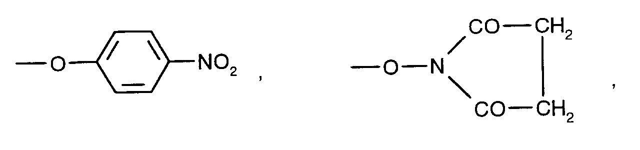 -NH-NH₂ oder -NH-NH₃^{⊕}Cl^{⊖}, 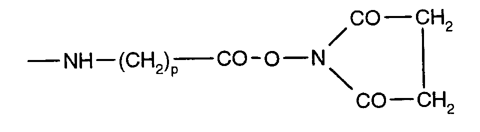 oder -NH-(CH₂-)ₚ-O-SO₂-R⁵ steht,
R⁵ CH₃, CₚF₂ₚ₊₁ oder Phenyl bedeutet,
R⁶ -(CH₂-)ₘ-CO-Y oder Y bedeutet,
m und n unabhängig voneinander ganze Zahlen von 1 bis 4 sind und
o und p unabhängig voneinander ganze Zahlen von 1 bis 9 sind.

3. Träger nach Anspruch 1, bei dem das Polymer für die monomolekulare Schicht nach b) im Falle der Anwendung der LB-Technik eines mit statistisch wiederkehrenden Einheiten der Formel 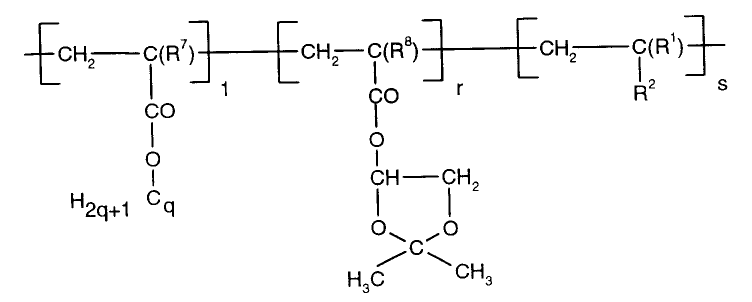 ist, in der
R¹ und R² die in Anspruch 2 genannte Bedeutung haben,
R⁷ und R⁸ unabhängig voneinander H oder CH₃ bedeuten,
q eine ganze Zahl von 12-20, bevorzugt 16-18 darstellt,
r einen Wert von 0,75-1,25, bevorzugt 0,8-1,1, besonders bevorzugt 0,9-1,05 bedeutet und
s einen Wert von 0,02-0,25, bevorzugt 0,05-0,2 bedeutet.

4. Träger nach Anspruch 1, bei dem das Polymer für die monomolekulare Schicht nach b) im Falle der Anwendung der SA-Technik eines mit statistisch wiederkehrenden Einheiten der Formel 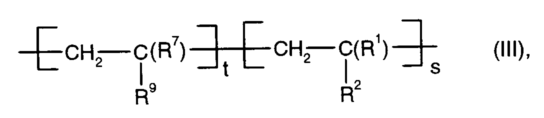 ist, in der
R¹, R², R³ und s die in Anspruch 3 gegebene Bedeutung haben,
t einen Wert von 1,6 bis 2,25, bevorzugt 1,8 bis 2,1, besonders bevorzugt 1,9 bis 2,05 annimmt und
R⁹ ein Anion aus der Gruppe von -SO₃^{⊖}, -C₆H₄-SO₃^{⊖}, -COO^{⊖} und -O-PO₃^{⊖⊖}
oder
ein Kation aus der Gruppe von 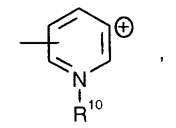 -N(R¹⁰, R¹¹, R¹²), -S(R¹⁰, R¹¹) , 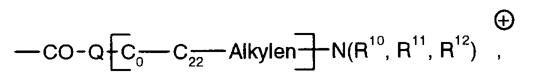 -NH-NH₃^{⊕} oder 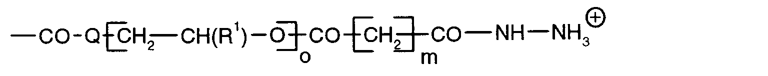 bedeutet,
worin R¹⁰, R¹¹und R¹² unabhängig voneinander Wasserstoff, C₁-C₂₂-Alkyl, Phenyl oder Benzyl bedeuten und
Q für O oder NH steht und die Indices o und m unabhängig voneinander ganze Zahlen von 1 bis 9 darstellen.

5. Träger nach Anspruch 1, bei dem die hydrophile Spacergruppe eine Kettenlänge von 5-30, bevorzugt 7-30, besonders bevorzugt 13-30 Atomen hat, wobei sowohl C-Atome als auch Heteroatome gezählt werden.

6. Träger nach Anspruch 1, bei dem die hydrophile Spacergruppe eine Oligoethylenoxidgruppe oder eine Oligopropylenoxidgruppe mit 5-9 Einheiten ist.

7. Träger nach Anspruch 2, bei dem die am Ende einer hydrophilen Spacergruppe sitzende Aktivgruppe Hydrazid, N-Hydroxysuccinimid, Nitrophenylester, Isothiocyanat, Iodcyanat, Iodacetamid, Maleimid oder Methylsulfonat, bevorzugt Hydrazid ist.

8. Verfahren zur Herstellung beschichteter Träger, dadurch gekennzeichnet, daß ein inerter Träger nach der Langmuir-Blodgett (LB)- oder nach der Self-Assembly (SA-)-Technik mit einer monomolekularen Schicht aus einem für diese Techniken geeigneten Polymer beschichtet wird, wobei dieses 0,8-20 Mol-%, bezogen auf die Molzahl aller Comonomere, an einem Comonomer enthält, das eine zur Bindung von Molekülen biologischen Ursprungs geeignete, am Ende einer hydrophilen Spacergruppe sitzende Aktivgruppe besitzt und wobei zwischen dem inerten Träger und der monomolekularen Schicht 0-200 monomolekulare Schichten eines für die LB- oder SA-Technik geeigneten Polymers ohne Aktivgruppen enthaltendes Comonomer angeordnet werden.

9. Verwendung des beschichteten Trägers nach Anspruch 1 zur Anbindung (Immobilisierung) von Molekülen biologischen Ursprungs.

10. Verwendung nach Anspruch 9, dadurch gekennzeichnet, daß zunächst Protein A oder Protein G und dann das betreffende Immunglobulin an die Oberfläche des Festkörpers gebunden wird.
